# EUROPEAN PATENT SPECIFICATION

(11) **EP 0 536 512 B1**
(45) Date of publication and mention of the grant of the patent: **05.11.1997**
(21) Application number: 92114142.0
(22) Date of filing: 19.08.1992
(51) Int. Cl.: C07D 275/04, C07C 323/22, C07D 451/02, C07D 211/32

(54) **A method and intermediates for the preparation of 6-fluorobenzisothiazoles**
Verfahren und Zwischenprodukte für die Herstellung von 6-Fluorbenzisothiazolen
Procédé et intermédiaire pour la préparation de 6-fluoro benzisothiazoles

(30) Priority: 22.08.1991 US 748729
(43) Date of publication of application: 14.04.1993
(73) Proprietor: HOECHST MARION ROUSSEL, Inc., Kansas City, Missouri 64137-1405 (US)
(72) Inventor: Fink, David M., Doylestown, PA 18901 (US); Strupczewski, Joseph T., Flemington, NJ 08822 (US)
(74) Representative: Losert, Wolfgang Dr.

(56) References cited:
- EP-A- 402 644
- EP-A- 0 454 621
- BERICHTE DER DEUTSCHEN CHEMISCHEN GESELLSCHAFT, vol. 56, no. 7, 4 July 1923, BERLIN, pages 1630 - 1633, K. FRIES ET AL.: 'Über Abkömmlinge des Benzo-4,5- isothiazols'

## Description

This invention relates to a process for the preparation of 6-fluoro-1,2-benzisothiazoles of the formula 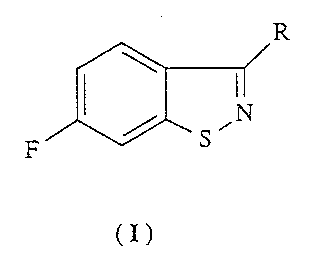 where R is hydrogen, (C₁-C₆)alkyl, cyclo(C₃-C₇)alkyl, ar(C₁-C₆)alkyl,aryl, where the term "aryl" means a phenyl group optionally substituted with 1, 2 or 3 substituents selected from: (C₁-C₆)alkyl,(C₁-C₆)alkoxy, halogen, hydroxy, trifluoromethyl, phenoxy and benzyloxy, or R is a nitrogen-containing heterocycloalkyl of the formula 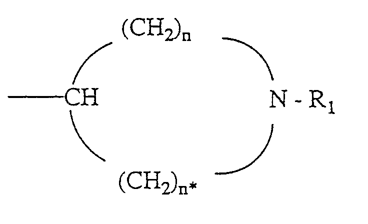 or bicycloheteroalkyl of the formula 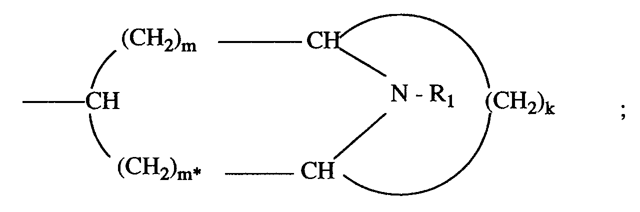 wherein R₁ is -CHO, -CO₂R₂, -CN or -C(=O)R₂; R₂ is (C₁-C₆)alkyl or aryl, where "aryl" is as defined above; k, m, m*, n and n* are independently integers from 1 to 4;
which process comprises reacting an *o*-halophenacyl compound of the formula 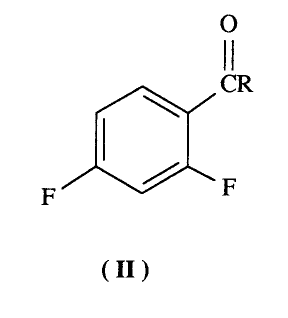 wherein R is as defined above, with R₃SH to obtain a sulfide compound of the formula 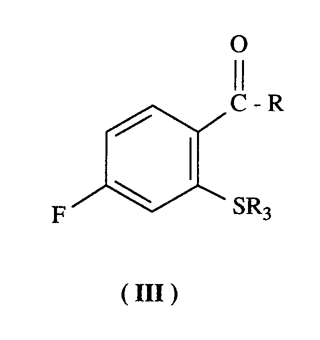 wherein R₃ is hydrogen, (C₁-C₆)alkyl or aryl(C₁-C₆)alkyl, where "aryl" is as defined above;
reacting the sulfide compound of Formula III with a halogenating agent to obtain the corresponding sulfenyl halide of the formula 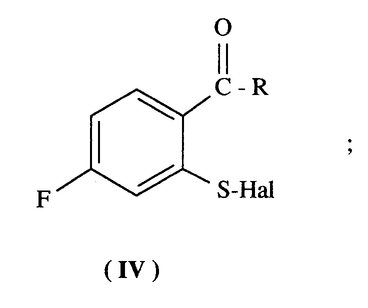 and
reacting the sulfenyl halide with ammonia to obtain a compound of the formula

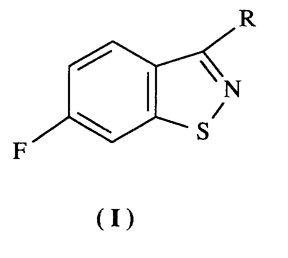

The resulting 1,2-benzisothiazoles (I) are useful as intermediates in the preparation of pharmaceutically active compounds, which are useful, for example, as antipsychotic agents and as inhibitors of the re-uptake of serotonin.

Additionally, this invention relates to compounds of the formula 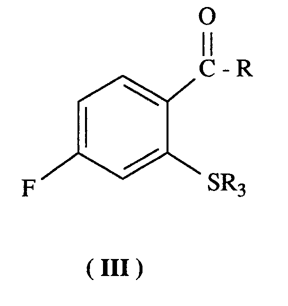 where R and R₃ are as previously defined, with the proviso that R is not hydrogen when R₃ is (C₁-C₆)alkyl or aryl(C₁-C₆)alkyl, which are useful as intermediates for the preparation of compounds of Formula IV.

In a preferred embodiment of the invention, R is hydrogen, (C₁-C₆)alkyl, 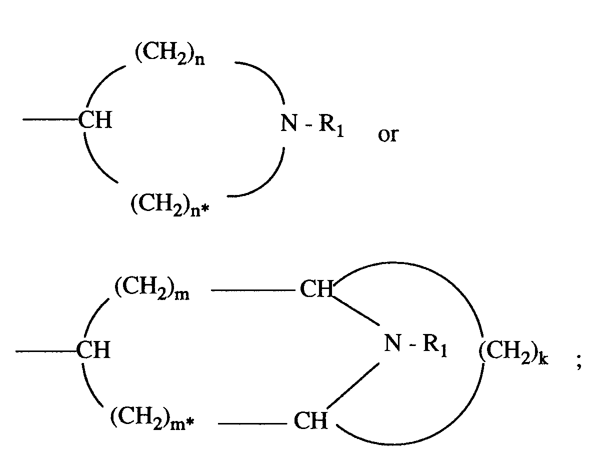 k, m, m*, n and n* are independently integers from 1 to 4; R₁ is -CHO, -CO₂R₂, -CN or 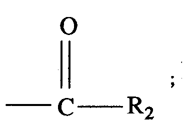 R₂ is (C₁-C₆)alkyl or aryl, where "aryl" is as defined above; and R₃ is benzyl. More preferably, R is 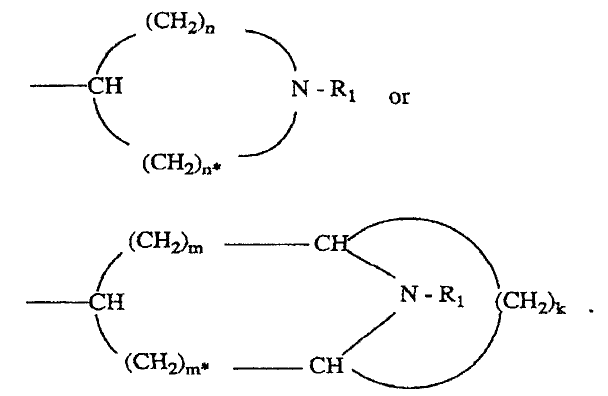 Most preferably, R is 4-piperidinyl or azabicyclo[3.2.1]octan-3-yl, R₁ is -CHO or -CN, and R₃ is benzyl.

Unless otherwise stated or indicated, the term (C₁-C₆)alkyl means a straight or branched alkyl group having from 1 to 6 carbon atoms. Examples of alkyl include methyl, ethyl, *n*-propyl, *iso*-butyl, pentyl, and hexyl.

Unless otherwise stated or indicated, the term cyclo(C₃-C₇)alkyl means a saturated ring containing 3 to 7 carbon atoms. Examples of cycloalkyl include cyclopropyl, cyclohexyl and cycloheptyl.

Unless otherwise stated or indicated, the term halogen means fluorine, chlorine, bromine or iodine.

Unless otherwise stated or indicated, the term aryl means an unsubstituted phenyl group or a phenyl group substituted with 1, 2 or 3 substituents each of which being independently (C₁-C₆)alkyl, (C₁-C₆)alkoxy, halogen, hydroxy, trifluoromethyl, phenoxy or benzyloxy.

It is known to prepare 1,2-benzisothiazoles starting from o-halophenacyl compounds. One example is the treatment of *o*-halophenacyl compounds with molecular sulfur and ammonia in an autoclave at high temperatures. However, it has not been possible to use such process to obtain 6-fluoro-1,2-benzisothiazoles in good yield.

The process of this invention provides an efficient means of preparing 6-fluoro-1,2-benzisothiazoles from o-halophenacyl compounds in good yield and without the need for nigh temperatures and autoclave conditions.

In the reaction to form compound III, it has been found that the displacement by the R₃SH nucleophile advantageously occurs selectively at the fluorine atom adjacent to the carbonyl functionality on the phenyl ring. The reaction is typically carried out in an organic solvent, for example, tetrahydrofuran, in the presence of base such as potassium *t*-butoxide at a temperature of from about 0°C to about 150°C, preferably from about 10°C to about 100°C, most preferably from about 15°C to about 80°C. Preferred R₃SH compounds include hydrogen sulfide, (C₁-C₆)alkyl mercaptans and benzyl mercaptans. Most preferred is benzyl mercaptan.

Compound III is reacted with a halogenating agent such as sulfuryl chloride, sulfuryl bromide, bromine or chlorine, most preferably with sulfuryl chloride, to obtain the sulfenyl halide IV. The reaction is generally carried out in an organic solvent such as dichloroethane or dichloromethane at a temperature of from about 0°C to about 100°C, preferably from about 15°C to about 80°C.

The resulting compound IV is then generally isolated and, without further purification, reacted with ammonia to obtain the desired benzisothiazole. The reaction is generally carried out in an organic solvent such as tetrahydrofuran at from about 0° C to about 100°C, preferably at from about 15°C to about 80°C, most preferably at about ambient temperature.

The starting difluoroketones wherein R is hydrogen, (C₁-C₆)alkyl, cyclo(C₃-C₇)alkyl, ar(C₁-C₆)alkyl or aryl, where aryl is as defined, are prepared according to procedures known in the art.

The starting difluoroketones wherein R is heterocyclo(C₃-C₇)alkyl such as piperidinyl are prepared for example by a Friedel-Crafts acylation of 1,3-difluorobenzene using the appropriate acid halide as is known in the art.

The starting difluoroketones wherein R is bicycloheteroalkyl such as tropanyl are prepared from 2,4-difluorobenzaldehyde dialkyl acetal which is reacted with a phosphorylating agent such as triethyl phosphite and with boron trifluoride etherate in a suitable solvent such as dichloromethane to afford Compound V of the formula 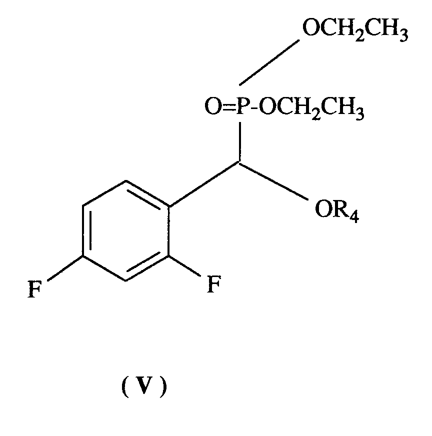 wherein R₄ is (C₁-C₆)alkyl. This reaction typically takes place at a temperature of -25°C to room temperature for 10 to 30 hours.

Compound V is reacted with *n*-butyllithium or other suitable agents, such as lithium diisopropyl amide, and tropinone of the formula 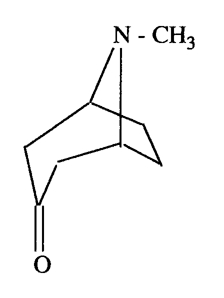 to afford Compound VI of the formula 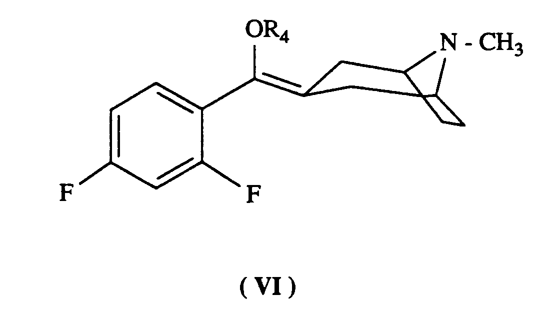 Typically, this reaction takes place in a suitable solvent such as tetrahydrofuran at a temperature of -78°C to room temperature for 10 to 30 hours.

Compound VI is reacted with aqueous HCl in acetone at reflux for 2 to 8 hours to afford Compound VII

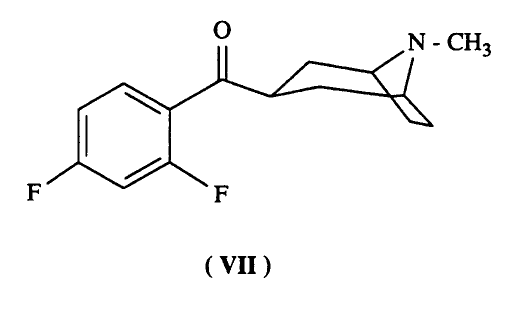

Compound VII is converted to Compound II where R₁ is CN or CO₂R_{2,}by means known in the art, for example, reaction with cyanogen bromide or a chloroformate in the presence of a base such as potassium carbonate yields Compound II wherein R₁ is CN or CO₂R₂ respectively.

The following examples are for illustration purposes only and are not to be construed as limiting the invention. All temperatures are given in degrees centigrade (°C) unless indicated otherwise.

### EXAMPLE 1

### a. 4-Fluoro-2-(phenylmethylthio)benzaldehyde

Bezyl mercaptan (1.74 g) in 10 mL of tetrahydrofuran (THF) was added dropwise to a solution of potassium t-butoxide (1.58 g) in 60 mL of THF. The resulting suspension was stirred at ambient temperature for 5 minutes, and then 2,4-difluorobenzaldehyde (2.0 g) was added and the reaction mixture was stirred at room temperature for one half hour. Saturated ammonium chloride solution was added, and the resulting mixture was extracted with ethyl acetate (EtOAc). The combined organic layers were washed with brine, dried over MgSO₄, filtered and evaporated to leave 3.11 g of crude product. Crystallization from methanol provided a solid, m.p. 81-82°C.

| ANALYSIS: | | |
|---|---|---|
| Calculated for C₁₄H₁₁FOS: | 68.27%C | 4.50%H |
| Found: | 68.31%C | 4.37%H |

### b. 6-Fluoro-1,2-benzisothiazole

Sulfuryl chloride (1.04 g) was added dropwise to a suspension of 4-fluoro-2-(phenylmethylthio)benzaldehyde (2.0 g) in 23 mL of dichloromethane at room temperature. The resulting solution was stirred for one half hour. Then the solvent was removed *in vacuo*, and the residue was suspended in 10 mL of THF and treated at room temperature with 10 mL of ethanol (EtOH) saturated with ammonia (NH₃). The resultant mixture was stirred for one half hour, water was then added, and the product was extracted into EtOAc. The combined organic layers were washed with brine, dried over MgSO₄, filtered and evaporated to leave 1.0 g of crude product. Purification by radially accelerated preparative thin layer chromatography (elution with ethyl acetate - hexanes) provided 0.66 g of a pale yellow liquid.

### EXAMPLE 2

### a. 4-Fluoro-2(phenylmethylthio)acetophenone

Benzyl mercaptan (1.59 g) in 10 mL of THF was added dropwise to a solution of potassium *t*-butoxide (1.4 g) in 55 mL of THF. The resulting suspension was stirred at ambient temperature for 5 minutes, and then 2,4-difluoroacetophenone (2.0 g) was added. The reaction mixture was stirred at room temperature for one half hour, saturated ammonium chloride solution was added, and the resulting mixture was extracted with EtOAc. The combined organic layers were washed with brine, dried over MgSO₄, filtered and evaporated to leave 3.2 g of crude product. Recrystallization from methanol gave 2.1 g (63%) of product as a solid, m.p. 111-112°C.

| ANALYSIS: | | |
|---|---|---|
| Calculated for C₁₅H₁₃FOS: | 69.21%C | 5.03%H |
| Found: | 69.42%C | 5.11%H |

### b. 6-Fluoro-3-methyl-1,2-benzisothiazole

Sulfuryl chloride (0.52 g) was added dropwise to a suspension of 4-fluoro-2-(phenylmethylthio)acetophenone (1.0 g) in 12 mL of dichloroethane at room temperature. The resulting solution was stirred for one half hour. Then the solvent was removed *in vacuo*, and the residue was suspended in 12 mL of THF and treated at room temperature with 12 mL of EtOH saturated with NH₃. The resultant mixture was stirred for one half hour, water was then added, and the product was extracted into EtOAc. The combined organic layers were washed with brine, dried over MgSO₄, filtered and evaporated to leave 0.6 g of crude product. Purification by radially accelerated preparative thin layer chromatography (elution with ethyl acetate - hexanes) provided 0.37 g (58%) of a pale yellow sold, m.p. 50-57°C.

### EXAMPLE 3

### a. 4-[4-Fluoro-2-(phenylmethylthio)benzoyl]-1-piperidinecarboxaldehyde

To a stirred solution of potassium *t*-butoxide (4.6 g) in THF (125 ml), was added, dropwise, benzyl mercaptan (5.1 g) in THF (60 ml). After stirring at ambient temperature for one half hour, 4-(2,4-difluorobenzoyl)-1-piperidinecarboxaldehyde (10.3 g) was added portionwise. The white flocculent solid that was present in the reaction went into solution, and stirring was continued at ambient temperature for two hours. The reaction was quenched into water, and the aqueous suspension extracted with EtOAc. The organic extract was washed (water), dried (MgSO₄), and the solvent was evaporated to afford 14.0 g of a viscous oil. The oil was combined with a sample from another run (14.7 g), and the combined sample triturated with EtO₂ to yield 20.4 g (63%) of a solid, m.p. 96-98°C. Recrystallization from toluene-hexane afforded an off-white solid, m.p. 101-103°C.

| ANALYSIS: | | | |
|---|---|---|---|
| Calculated for C₂₀H₂₀FNO₂S: | 67.20%C | 5.64%H | 3.92%N |
| Found: | 67.42%C | 5.96%H | 3.90%N |

### b. 4-(6-Fluoro-1,2-benzisothiazol-3-yl)-1-piperidinecarboxaldehyde

To a stirred solution of 4-[4-fluoro-2-(phenylmethylthio)]-1-piperidinecarboxaldehyde (40.0 g) in methylene chloride (CH₂Cl₂) (400 ml) was added, dropwise, sulfuryl chloride (8.9 ml) in CH₂Cl₂ (28 ml). After complete addition, the reaction was stirred at ambient temperature for one hour. The reaction was filtered to remove a small amount of insoluble material, and was then evaporated to afford 40.7 g of the sulfenyl chloride as a thick oil, which solidified upon standing. This crude sulfenyl chloride was dissolved in THF (500 ml) and a saturated solution of NH₃ in EtOH (180 ml) was added dropwise. The reaction was stirred at ambient temperature for two hours, and allowed to stand at ambient temperature for sixteen hours. The reaction mixture was poured into water, and the aqueous suspension was extracted with EtOAc. The organic extract was washed (water), dried (MgSO₄) and the solvent was evaporated to afford 29.6 g of a thick oil which was chromatographed on a preparative HPLC on silica gel columns, eluting with 3% Et₂NH-EtOAc to yield 12.2 g (42%) of compound as an oil. Trituration with isopropyl ether and scratching afforded a solid which was recrystailized from EtOAc-hexane to afford a solid, m.p. 104-106°C.

| ANALYSIS: | | | |
|---|---|---|---|
| Calculated for C₁₃H₁₃FN₂OS: | 59.07%C | 4.96%H | 10.60%N |
| Found: | 59.17%C | 5.07%H | 10.48%N |

### EXAMPLE 4

### a. Diethyl-1-(2,4-difluorophenyl)-1-methoxymethane phosphonate

Boron trifluoride etherate (86 g) was added dropwise to a solution of 2,4-difluorobenzaldehyde dimethyl acetal (114 g) and triethylphosphite (101 g) in 1.2 L of CH₂Cl₂ at -25°C. The resulting solution was allowed to warm to room temperature and stirred for twenty hours. Water was added, and the mixture was stirred vigorously for ten minutes. The organic layer was separated, washed with brine, dried over MgSO₄, filtered and concentrated to leave a liquid. Purification by HPLC on silica gel (elution with CH₂Cl₂, followed by 5% EtOAc-CH₂Cl₂) provided 136 g of diethyl-1-(2,4-difluorophenyl)-1-methoxymethane phosphonate, as a liquid.

### b. (2,4-Difluorophenyl)(8-methyl-8-azabicyclo[3.2.1]octan-3-yl)methanone hydrochloride

Diethyl-1-(2,4-difluorophenyl))-1-methoxymethane phosphonate (76 g) was dissolved in THF (1600 mL). The solution was cooled to -78°C and *n*-butyl lithium (103.4 mL of a 2.5 M solution in hexanes) was added dropwise at a rate such that the temperature never rose above -65°C. The resulting solution was stirred for one hour. Tropinone (32.7 g) dissolved in THF (100 mL) was then added slowly dropwise to the reaction mixture. After complete addition, the mixture was allowed to warm slowly to room temperature and was stirred overnight. A saturated NaCl solution (1.5 L) was added to the reaction mixture. The layers were separated and the organic layer was collected and dried over MgSO₄. The solvent was evaporated to yield an oil (73 g). This oil (38 g) was dissolved in acetone (2 L). Water (35 mL) and concentrated HCl (182 mL) were added slowly and the mixture was refluxed for three hours. The acetone was evaporated and the aqueous residue which remained was extracted with EtOAc, basified with K₂CO₃, extracted with CH₂Cl₂, and dried over MgSO₄. Evaporation of the solvent yielded 31.3 g of an oil which solidified. A portion of this solid (3 g) was dissolved in EtOH (75 mL). Ethanolic HCl was added until the solution was acidic. Ethyl ether (75 mL) was added and the product salt (2.65 g, m.p. 224-225°C) precipitated from solution.

| ANALYSIS: | | | |
|---|---|---|---|
| Calculated for C₁₅H₁₈ClF₂NO: | 59.70%C | 6.01%H | 4.64%N |
| Found: | 59.52%C | 5.87%H | 4.55%N |

### c. 3-(2,4-Difluorobenzoyl)-8-azabicyclo[3.2.1]octane-8-carbonitrile

Cyanogen bromide (4.0 g) was added in one portion to a suspension of (2,4-difluorophenyl)(8-methyl-8-azabicyclo[3.2.1]octan-3-yl)methanone (5.0 g) and potassium carbonate (5.2 g) in 80 mL of dimethylformamide at room temperature. The mixture was stirred for two hours, and then it was diluted with water and EtOAc. The layers were separated, and the aqueous phase was extracted with EtOAc. The combined organic layers were washed with water and brine, dried over MgSO₄, filtered and concentrated to leave 3.5 g of a solid which was recrystallized from EtOAc-heptane, m.p. 162-164°C.

| ANALYSIS: | | | |
|---|---|---|---|
| Calculated for C₁₅H₁₄F₂N₂O: | 65.21%C | 5.11%H | 10.14%N |
| Found: | 65.16%C | 5.10%H | 10.08%N |

### d. 3-[4-Fluoro-2-(phenylmethylthio)benzoyl]-8-azabicyclo[3.2.1]octane-8-carbonitrile

Benzyl mercaptan (0.67 g) in 5 mL of THF was added dropwise to a solution of potassium *t*-butoxide (0.6 g) in 15 mL of THF. The resulting suspension was stirred at ambient temperature for five minutes, and then (2,4-difluorobenzoyl)-8-azabicyclo[3.2.1]octane-8-carbonitrile (1.5 g) was added. The reaction mixture was stirred at room temperature for one half hour, then saturated ammonium chloride solution was added, and the product was extracted into EtOAc. The combined organic layers were washed with brine, dried over MgSO₄, filtered and concentrated to leave 1.7 g of crude product. Recrystallization from methanol gave 0.87 g of product as a solid, m.p. 166-167°C.

| ANALYSIS: | | | |
|---|---|---|---|
| Calculated for C₂₂H₂₁FN₂OS: | 69.45%C | 5.56%H | 7.36%N |
| Found: | 69.37%C | 5.69%H | 7.15%N |

### e. 3-(6-Fluoro-1,2-benzisothiazol-3-yl)-8-azabicyclo-[3.2.1]octane-8-carbonitrile

Sulfuryl chloride (5.33 g) was added dropwise to a suspension of 3-[4-fluoro-2-(phenylmethylthio)benzoyl]-8-azabicylo-[3.2.1]octane-8-carbonitrile (15 g) in 160 mL of dichloroethane at room temperature. The resulting solution was stirred for one and a quarter hours, and then the solvent was removed *in vacuo*, and the residue was suspended in 160 mL of THF and treated at room temperature with 160 mL of ethanol saturated with ammonia. The resultant mixture was stirred for 1.5 hours, and then the solvent was removed in vacuo and the residue was partitioned between water and CH₂Cl₂. The combined organic layers were washed with brine, dried over MgSO₄, filtered and evaporated to leave 10.7 g of a solid which was filtered through silica gel (elution with CH₂Cl₂). The solid obtained from evaporation of the filtrate (6.82 g) was recrystallized from EtOAc, providing 4.1 g of a white solid, m.p. 193-195°C.

| ANALYSIS: | | | |
|---|---|---|---|
| Calculated for C₁₅H₁₄FN₃S: | 62.70%C | 4.91%H | 14.62%N |
| Found: | 62.63%C | 4.90%H | 14.73%N |

It is to be understood that changes and variations may be made without departing from the spirit and scope of the invention as defined by the appended claims.

## Claims (Claims for the following Contracting State(s): AT, BE, CH, DE, DK, FR, GB, GR, IE, IT, LI, LU, MC, NL, PT, SE)

1. A process for the preparation of 6-fluoro-1,2-benzisothiazoles of formula I 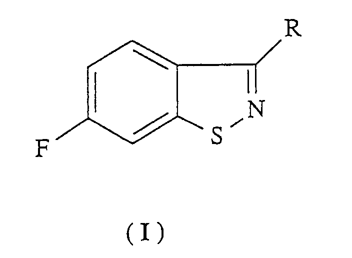 where R is hydrogen, (C₁-C₆)alkyl, cyclo(C₃-C₇)alkyl, ar(C₁-C₆)alkyl, aryl, where the term "aryl" means a phenyl group optionally substituted with 1, 2 or 3 substituents selected from: (C₁-C₆)alkyl, (C₁-C₆)alkoxy, halogen, hydroxy, trifluoromethyl, phenoxy and benzyloxy, or R is a group of the formulae 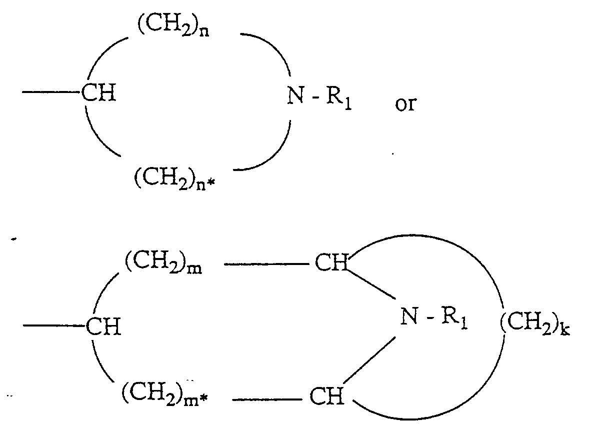 wherein R₁ is -CHO, -CO₂R₂, -CN or -C(=O)R_{2;} R₂ is (C₁-C₆)alkyl or aryl, where "aryl" is as defined above; k, m, m*, n and n* are independently integers from 1 to 4;
which comprises
a) reacting a compound of the formula III 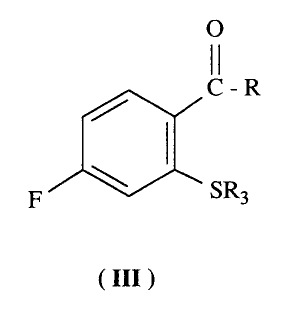 wherein R is as defined above and R₃ is hydrogen, (C₁-C₆)alkyl, or aryl(C₁-C₆)alkyl, where "aryl" is as defined above, with a halogenating agent to obtain a compound of the formula IV 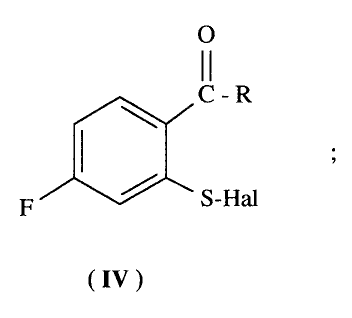 and wherein R is as defined and Hal is halogen, and
b) reacting the compound of formula IV as obtained in step a) with ammonia.

2. A process as claimed in claim 1, wherein R is hydrogen, (C₁-C₆)alkyl or a group of the formulae 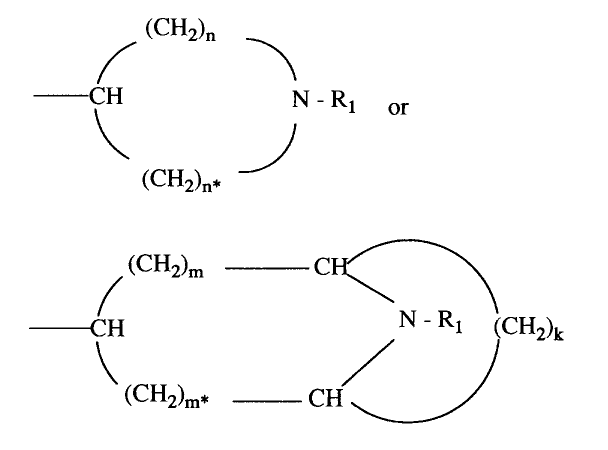 where k, m, m*, n, n*, R₁ and R₂ are as defined in claim 1 and R₃ is benzyl.

3. A process as claimed in claim 2, wherein R is a group of the formulae 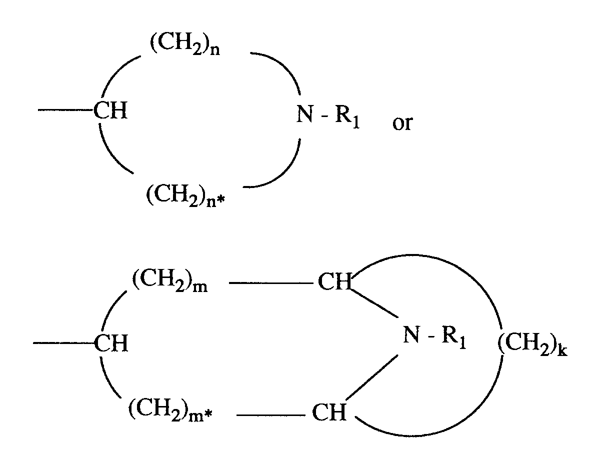 where k, m, m*, n, n*, R₁ and R₂ are as defined.

4. A process as claimed in claim 3, wherein n and n* are both the integer 2, m and m* are both the integer 1, k is the integer 2, and R₁ is -CHO or -CN.

5. The process as claimed in claim 1, wherein the compound of formula III is reacted with sulfuryl chloride, sulfuryl bromide, bromine or chlorine as the halogenating agent at a temperature of from 0°C to 100°C.

6. The process as claimed in claim 5, wherein the halogenating agent is sulfuryl chloride and the reaction temperature is from 15°C to 80°C.

7. The process as claimed in claim 1, wherein the compound of formula IV is reacted with ammonia at a temperature of from 0°C to 100°C.

8. The process as claimed in claim 7, wherein the reaction temperature is from 15°C to 80°C.

9. The process as claimed in claim 8, wherein the reaction temperature is ambient temperature.

10. A compound of the formula III 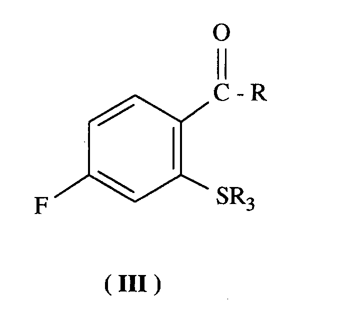 wherein R is hydrogen, (C₁-C₆)alkyl, cyclo(C₃-C₇)alkyl, ar(C₁-C₆)alkyl, aryl where the term "aryl" means a phenyl group optionally substituted with 1, 2 or 3 substituents selected from: (C₁-C₆)alkyl, (C₁-C₆)alkoxy, halogen, hydroxy, trifluoromethyl, phenoxy and benzyloxy, or R is a group of the formulae 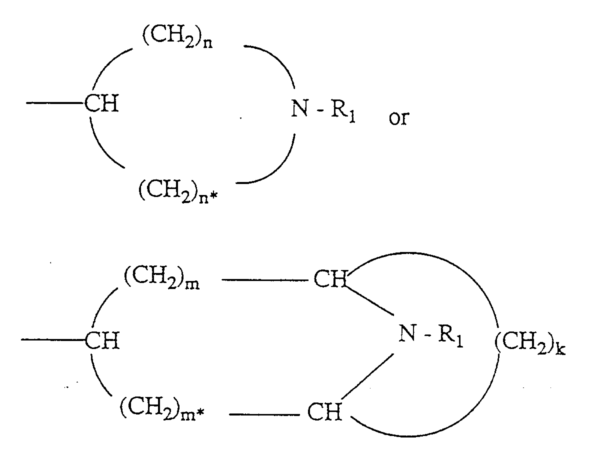 wherein R₁ is -CHO, -CO₂R₂, -CN or -C(=O)-R₂, where R₂ is (C₁-C₆)alkyl or aryl, where "aryl" is as defined above k, m, m*, n and n* are independently integers from 1 to 4 and R₃ is hydrogen, (C₁-C₆)alkyl or aryl(C₁-C₆)alkyl, where "aryl" is as defined above, with the proviso that R is not hydrogen when R₃ is (C₁-C₆)alkyl or aryl(C₁-C₆)alkyl.

11. A compound as claimed in claim 10, wherein R is hydrogen, (C₁-C₆)alkyl or a group of the formulae 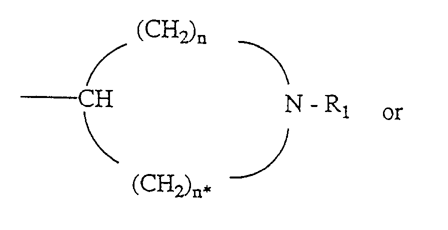 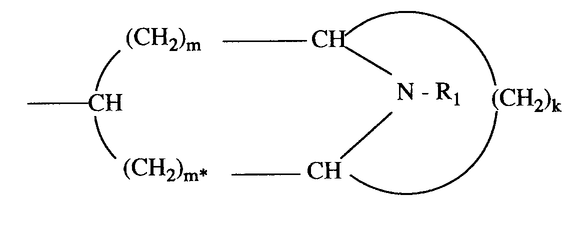 and R₃ is benzyl.

12. A compound as claimed in claim 11, wherein n and n* are both the integer 2, m and m* are both the integer 1, k is the integer 2 and R₁ is -CHO or -CN.

13. A process for the preparation of a compound as defined in claim 10, which comprises reacting a 2,4-difluorophenacyl compound of formula II 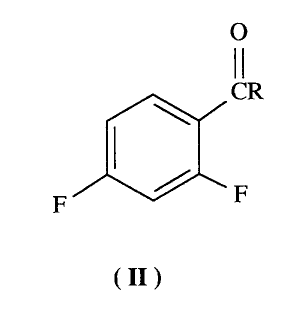 wherein R is as defined, with R₃SH, wherein R₃ is as defined, with the proviso that R is not hydrogen, when R₃ is (C₁-C₆)alkyl or aryl(C₁-C₆)alkyl.

## Claims (Claims for the following Contracting State(s): ES)

1. A process for the preparation of 6-fluoro-1,2-benzisothiazoles of formula I 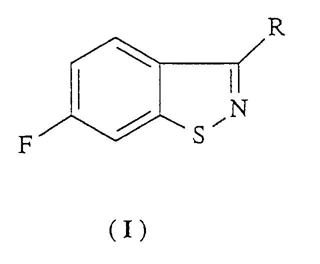 where R is hydrogen, (C₁-C₆)alkyl, cyclo(C₃-C₇)alkyl, ar(C₁-C₆)alkyl, aryl, where the term "aryl" means a phenyl group optionally substituted with 1, 2 or 3 substituents selected from: (C₁-C₆)alkyl, (C₁-C₆)alkoxy, halogen, hydroxy, trifluoromethyl, phenoxy and benzyloxy, or R is a group of the formulae 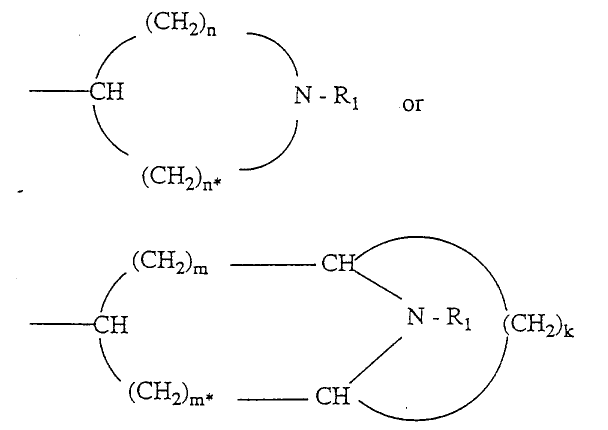 wherein R₁ is -CHO, -CO₂R₂, -CN or -C(=O)R₂; R₂ is (C₁-C₆)alkyl or aryl, where "aryl" is as defined above; k, m, m*, n and n* are independently integers from 1 to 4;
which comprises
a) reacting a compound of the formula III 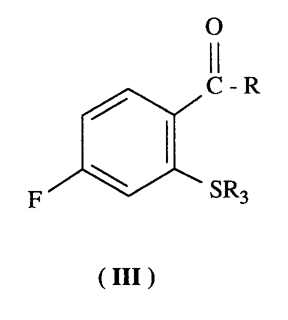 wherein R is as defined above and R₃ is hydrogen, (C₁-C₆)alkyl, or aryl(C₁-C₆)alkyl, where "aryl" is as defined above, with a halogenating agent to obtain a compound of the formula IV 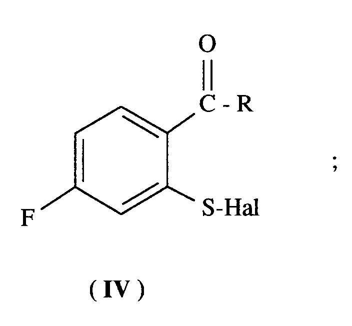 and wherein R is as defined and Hal is halogen, and
b) reacting the compound of formula IV as obtained in step a) with ammonia.

2. A process as claimed in claim 1, wherein R is hydrogen, (C₁-C₆)alkyl or a group of the formulae 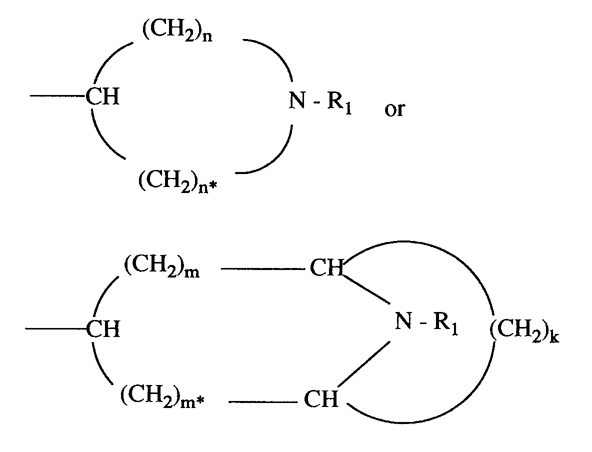 where k, m, m*, n, n*, R₁ and R₂ are as defined in claim 1 and R₃ is benzyl.

3. A process as claimed in claim 2, wherein R is a group of the formulae 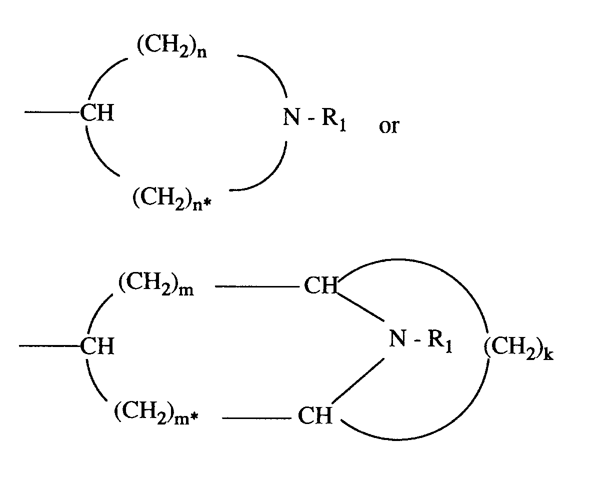 where k, m, m*, n, n*, R₁ and R₂ are as defined.

4. A process as claimed in claim 3, wherein n and n* are both the integer 2, m and m* are both the integer 1, k is the integer 2, and R₁ is -CHO or -CN.

5. The process as claimed in claim 1, wherein the compound of formula III is reacted with sulfuryl chloride, sulfuryl bromide, bromine or chlorine as the halogenating agent at a temperature of from 0°C to 100°C.

6. The process as claimed in claim 5, wherein the halogenating agent is sulfuryl chloride and the reaction temperature is from 15°C to 80°C.

7. The process as claimed in claim 1, wherein the compound of formula IV is reacted with ammonia at a temperature of from 0°C to 100°C.

8. The process as claimed in claim 7, wherein the reaction temperature is from 15°C to 80°C.

9. The process as claimed in claim 8, wherein the reaction temperature is ambient temperature.

10. A process for the preparation of a compound of the formula III 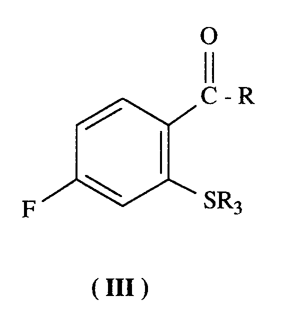 wherein R is hydrogen, (C₁-C₆)alkyl, cyclo(C₃-C₇)alkyl, ar(C₁-C₆)alkyl, aryl, where the term "aryl" means a phenyl group optionally substituted with 1, 2 or 3 substituents selected from: (C₁-C₆)alkyl, (C₁-C₆)alkoxy, halogen, hydroxy, trifluoromethyl, phenoxy and benzyloxy, or R is a group of the formulae: 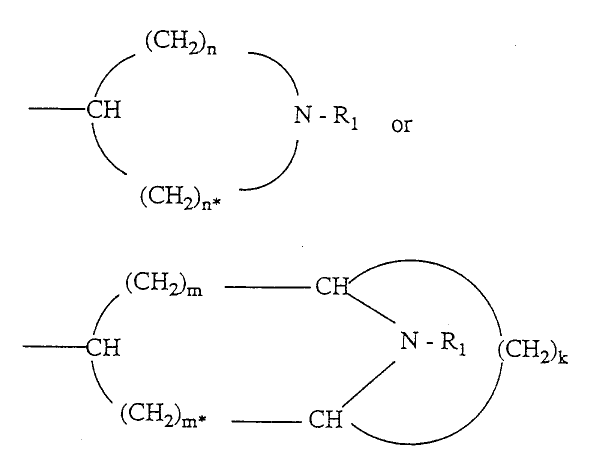 wherein R₁ is -CHO, -CO₂R₂, -CN or -C(=O)-R₂, where R₂ is (C₁-C₆)alkyl or aryl, where "aryl" is as defined above, k, m, m*, n and n* are independently integers from 1 to 4 and R₃ is hydrogen, (C₁-C₆)alkyl or aryl(C₁-C₆)alkyl, where "aryl" is as defined, which comprises reacting a 2,4-difluorophenacyl compound of the formula II 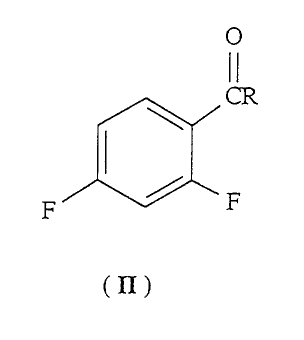 wherein R is as defined, with R₃SH, wherein R₃ is as defined, with the proviso that R is not hydrogen when R₃ is (C₁-C₆)alkyl or aryl(C₁-C₆)alkyl.

11. A process as claimed in claim 10, wherein R is hydrogen, (C₁-C₆)alkyl or a group of the formulae 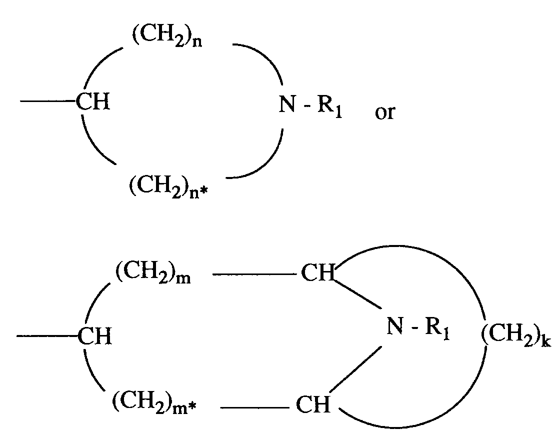 and R₃ is benzyl.

12. A process as claimed in claim 11, wherein n and n* are both the integer 2, m and m* are both the integer 1, k is the integer 2 and R₁ is -CHO or -CN.

## Patentansprüche (Patentansprüche für folgende(n) Vertragsstaat(en): AT, BE, CH, DE, DK, FR, GB, GR, IE, IT, LI, LU, MC, NL, PT, SE)

1. Verfahren zur Herstellung von 6-Fluor-1,2-benzisothiazolen der Formel I 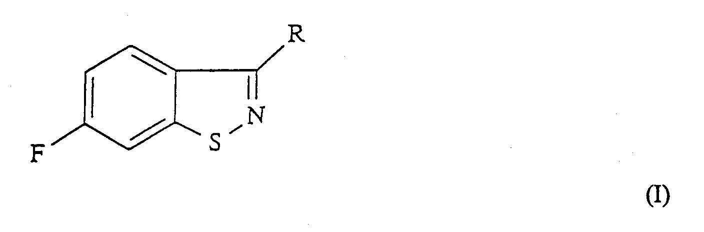 in welcher R für Wasserstoff, (C₁-C₆)-Alkyl, Cyclo-(C₃-C₇)-alkyl, Ar-(C₁-C₆)-alkyl, Aryl steht, wobei der Begriff "Aryl" eine Phenylgruppe bezeichnet, die wahlweise mit 1, 2 oder 3 Substituenten, ausgewählt aus: (C₁-C₆)Alkyl, (C₁-C₆)Alkoxy, Halogen, Hydroxy, Trifluormethyl, Phenoxy und Benzyloxy, substituiert ist, oder R eine Gruppe der Formeln 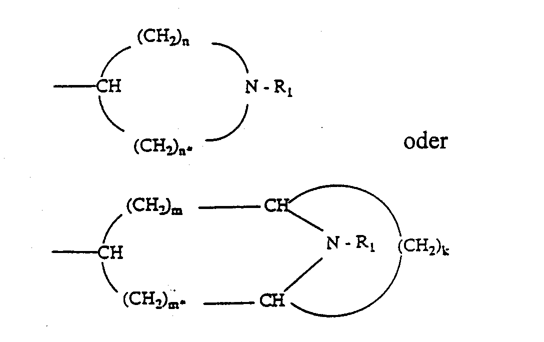 ist, wobei R₁ für -CHO, -CO₂R₂, -CN oder -C(=O)R₂ steht; R₂ (C₁-C₆)-Alkyl oder Aryl ist, wobei "Aryl" die obengenannte Bedeutung hat; k, m, m*, n und n* unabhängig voneinander ganze Zahlen von 1 bis 4 sind;
umfassend
a) die Umsetzung einer Verbindung der Formel III 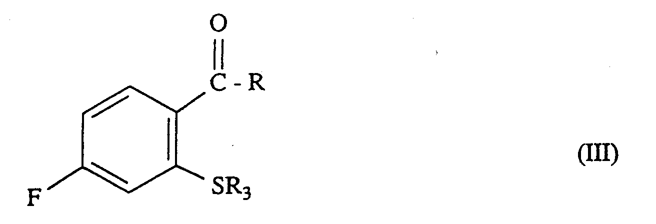 in der R die oben angegebene Bedeutung hat und R₃ Wasserstoff, (C₁-C₆)-Alkyl oder Aryl-(C₁-C₆)-alkyl ist, wobei "Aryl" die obengenannte Bedeutung hat, mit einem Halogenierungsmittel zur Herstellung einer Verbindung der Formel IV 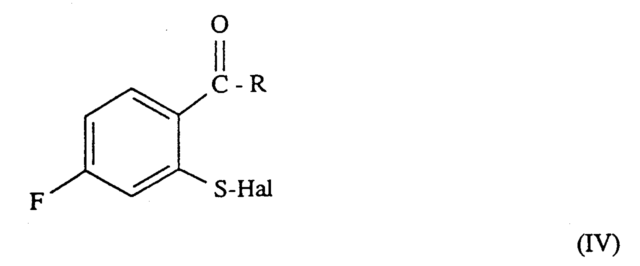 in der R die genannte Bedeutung hat und Hal für Halogen steht, und
b) die Umsetzung der wie in Abschnitt a) hergestellten Verbindung der Formel IV mit Ammoniak.

2. Verfahren gemäß Anspruch 1, in dem R Wasserstoff, (C₁-C₆)-Alkyl oder eine Gruppe der Formeln 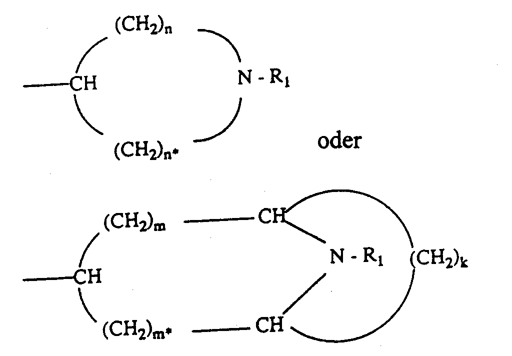 darstellt, wobei k, m, m*, n, n*, R₁ und R₂ die in Anspruch 1 angegebene Bedeutung haben, und R₃ Benzyl ist.

3. Verfahren gemäß Anspruch 2, in dem R eine Gruppe der Formeln 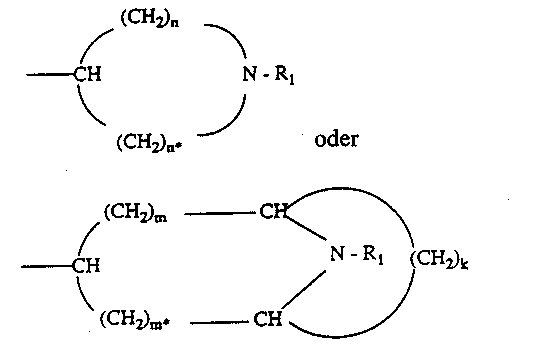 darstellt, wobei k, m, m*, n, n*, R₁ und R₂ die zuvor angewiesene Bedeutung zukommt.

4. Verfahren gemäß Anspruch 3, in dem n und n* beide die ganze Zahl 2, m und m* beide die ganze Zahl 1, k die ganze Zahl 2 und R₁ -CHO oder -CN sind.

5. Verfahren gemäß Anspruch 1, in dem die Verbindung der Formel III mit Sulfurylchlorid, Sulfurylbromid, Brom oder Chlor als Halogenierungsmittel bei einer Temperatur von 0°C bis 100°C umgesetzt wird.

6. Verfahren gemäß Anspruch 5, in dem das Halogenierungsmittel Sulfurylchlorid ist und die Reaktionstemperatur von 15°C bis 80°C beträgt.

7. Verfahren gemäß Anspruch 1, in dem die Verbindung der Formel IV bei einer Temperatur von 0°C bis 100°C mit Ammoniak umgesetzt wird.

8. Verfahren gemäß Anspruch 7, in dem die Reaktionstemperatur von 15°C bis 80°C beträgt.

9. Verfahren gemäß Anspruch 8, in dem die Reaktionstemperatur gleich der Umgebungstemperatur ist.

10. Verbindung der Formel III 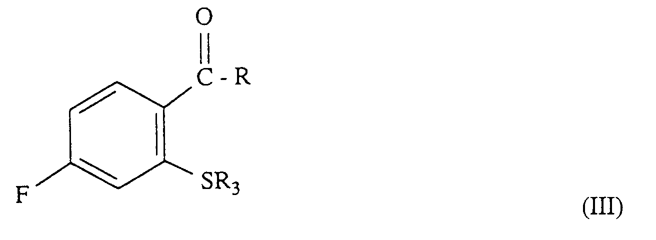 in welcher R für Wasserstoff, (C₁-C₆)-Alkyl, Cyclo-(C₃-C₇)-alkyl, Ar-(C₁-C₆)-alkyl, Aryl steht, wobei der Begriff "Aryl" eine Phenylgruppe bezeichnet, die wahlweise mit 1, 2 oder 3 Substituenten, ausgewählt aus: (C₁-C₆)-Alkyl, (C₁-C₆)-Alkoxy, Halogen, Hydroxy, Trifluormethyl, Phenoxy und Benzyloxy, substituiert ist, oder R eine Gruppe der Formeln 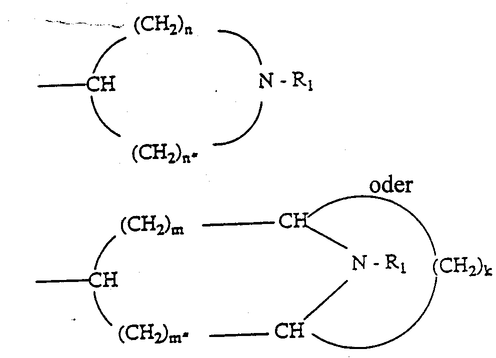 ist, wobei R₁ für -CHO, -CO₂R₂, -CN oder -C(=O)-R₂ steht; in welcher R₂ (C₁-C₆)-Alkyl oder Aryl ist, wobei "Aryl" die obengenannte Bedeutung hat; k, m, m*, n und n* unabhängig voneinander ganze Zahlen von 1 bis 4 sind und R₃ für Wasserstoff, (C₁-C₆)-Alkyl oder Aryl-(C₁-C₆)-alkyl steht, wobei "Aryl" die obengenannte Bedeutung hat, unter der Bedingung, daß R nicht Wasserstoff ist, wenn R₃ (C₁-C₆)-Alkyl oder Ary-l(C₁-C₆)alkyl ist.

11. Verbindung gemäß Anspruch 10, in der R Wasserstoff, (C₁-C₆)-Alkyl oder eine Gruppe der Formeln 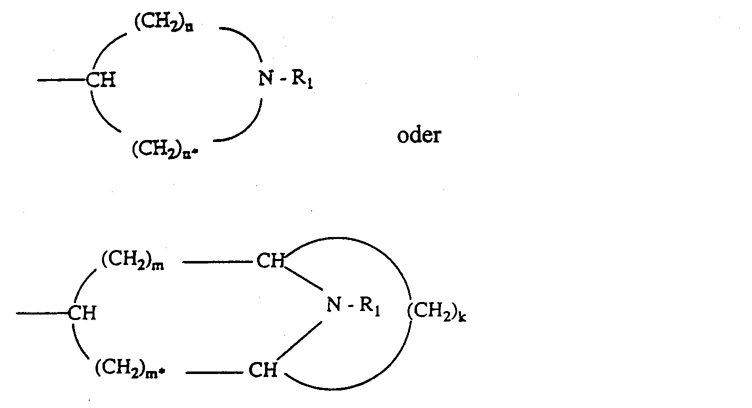 darstellt und R₃ Benzyl ist.

12. Verbindung gemäß Anspruch 11, in der n und n* beide die ganze Zahl 2, m und m* beide die ganze Zahl 1, k die ganze Zahl 2 und R₁ -CHO oder -CN sind.

13. Verfahren zur Herstellung einerVerbindung gemäß Anspruch 10, umfassend die Umsetzung einer 2,4-Difluorphenacyl-Verbindung der Formel II 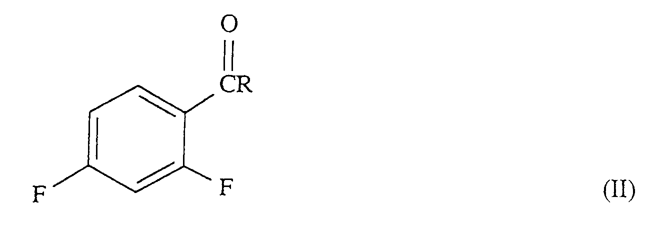 in der R die genannte Bedeutung hat, mit R₃SH, wobei R₃ die angegebene Bedeutung zukommt, unter der Bedingung, daß R nicht Wasserstoff ist, wenn R₃ (C₁-C₆)-Alkyl oder Aryl-(C₁-C₆)-alkyl ist.

## Patentansprüche (Patentansprüche für folgende(n) Vertragsstaat(en): ES)

1. Verfahren zur Herstellung von 6-Fluor-1,2-benzisothiazolen der Formel I 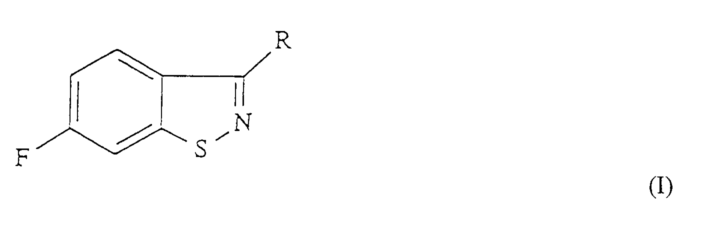 in welcher R für Wasserstoff, (C₁-C₆)-Alkyl, Cyclo-(C₃-C₇)-alkyl, Ar-(C₁-C₆)-alkyl, Aryl steht, wobei der Begriff "Aryl" eine Phenylgruppe bezeichnet, die wahlweise mit 1, 2 oder 3 Substituenten, ausgewählt aus: (C₁-C₆)Alkyl, (C₁-C₆)Alkoxy, Halogen, Hydroxy, Trifluormethyl, Phenoxy und Benzyloxy, substituiert ist, oder R eine Gruppe der Formeln 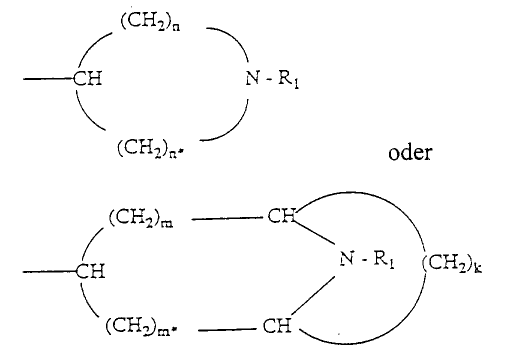 ist, wobei R₁ für -CHO, -CO₂R₂, -CN oder -C(=O)R₂ steht; R₂ (C₁-C₆)-Alkyl oder Aryl ist, wobei "Aryl" die obengenannte Bedeutung hat; k, m, m*, n und n* unabhängig voneinander ganze Zahlen von 1 bis 4 sind;
umfassend
a) die Umsetzung einer Verbindung der Formel III 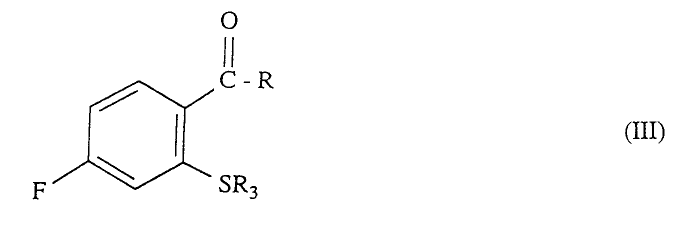 in der R die oben angegeben Bedeutung hat und R₃ Wasserstoff, (C₁-C₆)-Alkyl oder Aryl-(C₁-C₆)-alkyl ist, wobei "Aryl" die obengenannte Bedeutung hat, mit einem Halogenierungsmittel zur Herstellung einer Verbindung der Formel IV 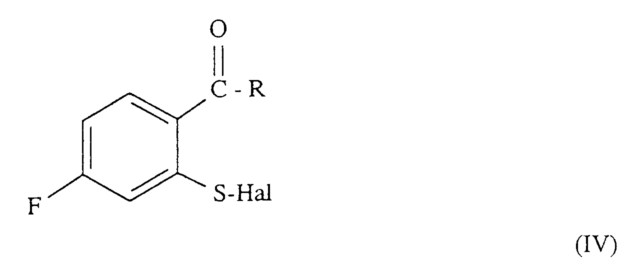 in der R die genannte Bedeutung hat und Hal für Halogen steht, und
b) die Umsetzung der wie in Abschnitt a) hergestellten Verbindung der Formel IV mit Ammoniak.

2. Verfahren gemäß Anspruch 1, in dem R Wasserstoff, (C₁-C₆)-Alkyl oder eine Gruppe der Formeln 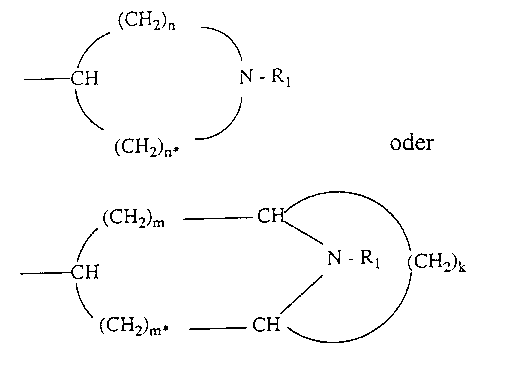 darstellt, wobei k, m, m*, n, n*, R₁ und R₂ die in Anspruch 1 angegebene Bedeutung haben, und R₃ Benzyl ist.

3. Verfahren gemäß Anspruch 2, in dem R eine Gruppe der Formeln 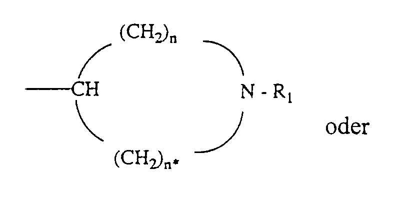 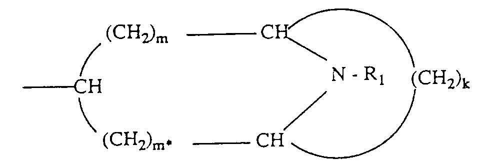 darstellt, wobei k, m, m*, n, n*, R₁ und R₂ die zuvor angewiesene Bedeutung zukommt.

4. Verfahren gemäß Anspruch 3, in dem n und n* beide die ganze Zahl 2, m und m* beide die ganze Zahl 1, k die ganze Zahl 2 und R₁ -CHO oder -CN sind.

5. Verfahren gemäß Anspruch 1, in dem die Verbindung der Formel III mit Sulfurylchlorid, Sulfurylbromid, Brom oder Chlor als Halogenierungsmittel bei einer Temperatur von 0°C bis 100°C umgesetzt wird.

6. Verfahren gemäß Anspruch 5, in dem das Halogenierungsmittel Sulfurylchlorid ist und die Reaktionstemperatur von 15°C bis 80°C beträgt.

7. Verfahren gemäß Anspruch 1, in dem die Verbindung der Formel IV bei einer Temperatur von 0°C bis 100°C mit Ammoniak umgesetzt wird.

8. Verfahren gemäß Anspruch 7, in dem die Reaktionstemperatur von 15°C bis 80°C beträgt.

9. Verfahren gemäß Anspruch 8, in dem die Reaktionstemperatur gleich der Umgebungstemperatur ist.

10. Verbindung der Formel III 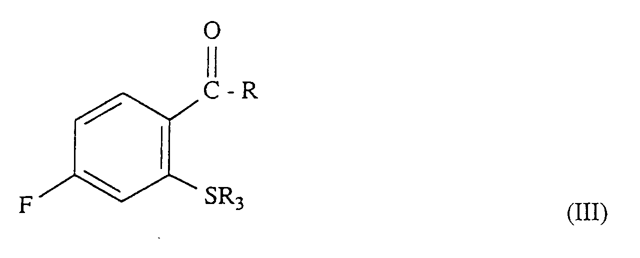 in welcher R für Wasserstoff, (C₁-C₆)-Alkyl, Cyclo-(C₃-C₇)-alkyl, Ar-(C₁-C₆)-alkyl, Aryl steht, wobei der Begriff "Aryl" eine Phenylgruppe bezeichnet, die wahlweise mit 1,2 oder 3 Substituenten, ausgewählt aus: (C₁-C₆)-Alkyl, (C₁-C₆)-Alkoxy, Halogen, Hydroxy, Trifluormethyl, Phenoxy und Benzyloxy, substituiert ist, oder R eine Gruppe der Formeln 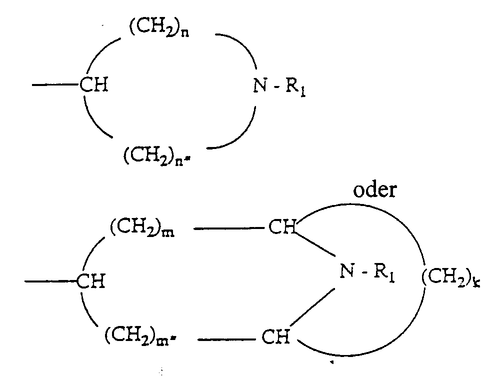 ist, wobei R₁ für -CHO, -CO₂R₂, -CN oder -C(=O)-R₂ steht; in welcher R₂ (C₁-C₆)-Alkyl oder Aryl ist, wobei "Aryl" die obengenannte Bedeutung hat; k, m, m*, n und n* unabhängig voneinander ganze Zahlen von 1 bis 4 sind und R₃ für Wasserstoff, (C₁-C₆)-Alkyl oder Aryl-(C₁-C₆)-alkyl steht, wobei "Aryl" die obengenannte Bedeutung hat, umfassend die Umsetzung einer 2,4-Difluorphenacyl-Verbindung der Formel II 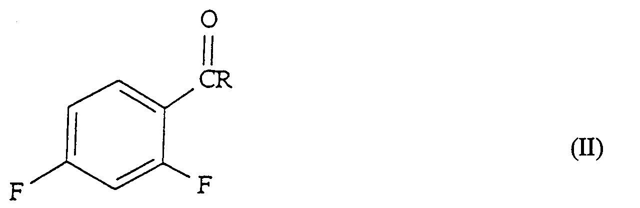 in der R die genannte Bedeutung hat, mit R₃SH, wobei R₃ die angegebene Bedeutung zukommt, unter der Bedingung, daß R nicht Wasserstoff ist, wenn R₃ (C₁-C₆)-Alkyl oder Aryl-(C₁-C₆)-alkyl ist.

11. Verfahren gemäß Anspruch 10, in dem R Wasserstoff, (C₁-C₆)-Alkyl oder eine Gruppe der Formeln 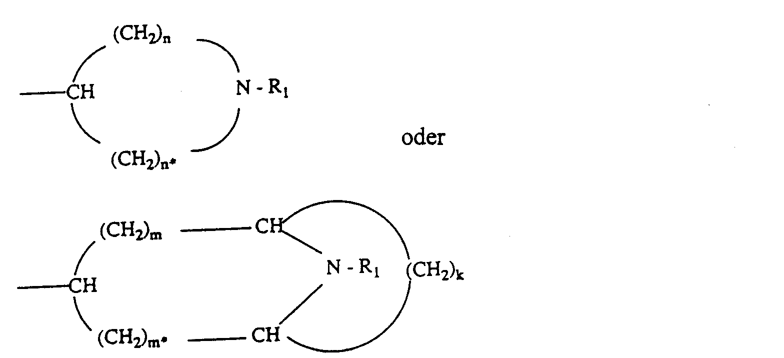 darstellt und R₃ Benzyl ist.

12. Verfahren gemäß Anspruch 11, in dem n und n* beide die ganze Zahl 2, m und m* beide die ganze Zahl 1, k die ganze Zahl 2 und R₁ -CHO oder -CN sind.

## Revendications (Revendications pour l'(les) Etat(s) contractant(s) suivant(s): AT, BE, CH, DE, DK, FR, GB, GR, IE, IT, LI, LU, MC, NL, PT, SE)

1. Procédé pour la préparation de 6-fluoro-1,2-benzisothiazoles de formule I 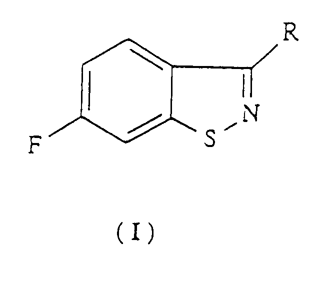 dans laquelle R représente un atome d'hydrogène, un groupe alkyle en C₁-C₆, cycloalkyle en C₃-C₇, aralkyle en C₁-C₆, aryle, le terme aryle représentant un groupe phényle éventuellement substitué avec 1, 2 ou 3 substituants choisis parmi : un groupe alkyle en C₁-C₆, alkoxy en C₁-C₆, un atome d'halogène, un groupe hydroxy, trifluorométhyle, phénoxy et benzyloxy, ou R représente un groupe de formule 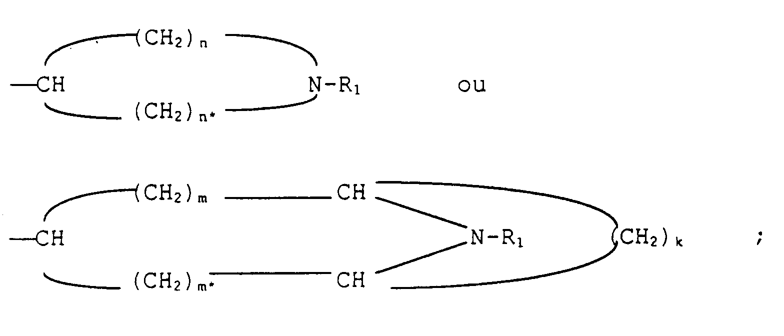 dans laquelle R₁ représente un groupe -CHO, -CO₂R₂, -CN ou -C(=O)R₂; R₂ représente un groupe alkyle en C₁-C₆ ou aryle, le terme "aryle" étant tel que défini ci-dessus; k, m, m*, n et n* étant indépendamment des entiers compris entre 1 et 4;
lequel procédé comprend
a) l'étape consistant à faire réagir un composé de formule III 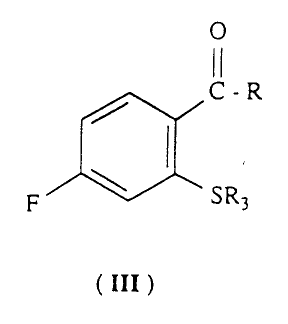 dans laquelle R est tel que défini ci-dessus et R₃ est un atome d'hydrogène, un groupe alkyle en C₁-C₆ ou aryle-alkyle en C₁-C₆, le terme "aryle" étant tel que défini ci-dessus, avec un agent d'halogénation pour obtenir un composé de formule IV 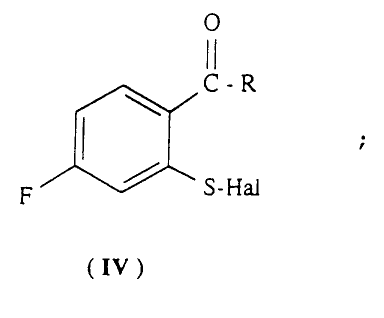 et dans laquelle R est tel que défini et Hal représente un atome d'halogène, et
b) l'étape consistant à faire réagir le composé de formule IV tel qu'obtenu à l'étape a) avec l'ammoniac.

2. Procédé selon la revendication 1, dans lequel R est un atome d'hydrogène, un groupe alkyle en C₁-C₆ ou un groupe de formule 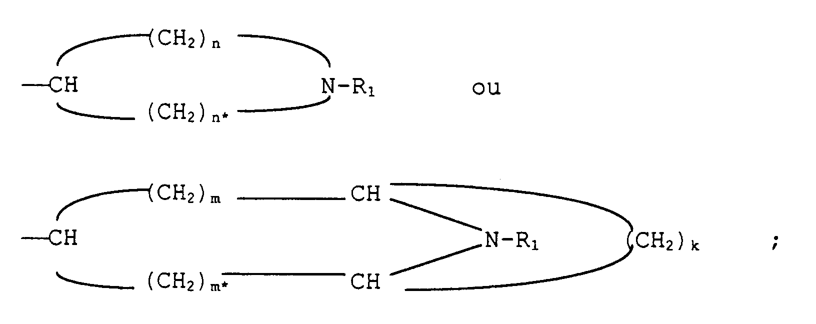 dans laquelle k, m, m*, n, n*, R₁ et R₂ sont tels que définis et R₃ est un groupe benzyle.

3. Procédé selon la revendication 2, dans lequel R est un groupe de formule 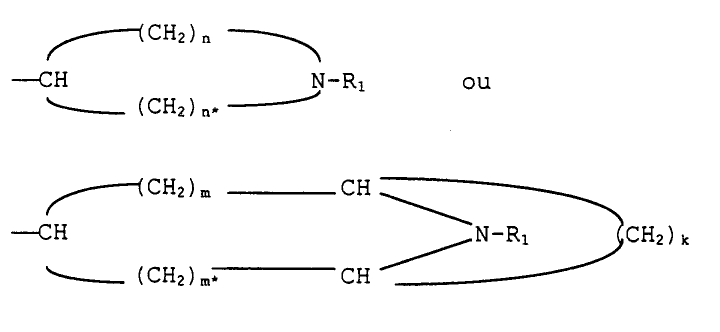 dans laquelle k, m, m*, n, n*, R₁ et R₂ sont tels que définis.

4. Procédé selon la revendication 3, dans lequel n et n* sont tous les deux l'entier 2, m et m* sont tous les deux l'entier 1, k est l'entier 2 et R₁ est un groupe -CHO ou -CN.

5. Procédé selon la revendication 1, dans lequel on fait réagir le composé de formule III avec le chlorure de sulfuryle, le bromure de sulfuryle, le brome ou le chlore comme agent d'halogénation à une température comprise entre 0°C et 100°C.

6. Procédé selon la revendication 5, dans lequel l'agent d'halogénation est le chlorure de sulfuryle et la température de réaction est comprise entre 15°C et 80°C.

7. Procédé selon la revendication 1, dans lequel on fait réagir le composé de formule IV avec l'ammoniac à une température comprise entre 0°C et 100°C.

8. Procédé selon la revendication 7, dans lequel la température de réaction est comprise entre 15°C et 80°C.

9. Procédé selon la revendication 8, dans lequel la température de réaction est la température ambiante.

10. Composé de formule III 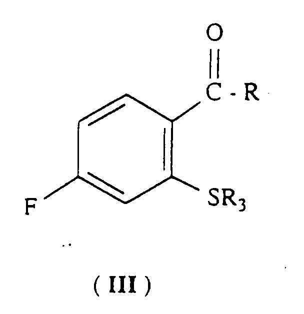 dans laquelle R représente un atome d'hydrogène, un groupe alkyle en C₁-C₆, cycloalkyle en C₃-C₇, aralkyle en C₁-C₆, aryle, le terme aryle représentant un groupe phényle éventuellement substitué avec 1, 2 ou 3 substituants choisis parmi : un groupe alkyle en C₁-C₆, alkoxy en C₁-C₆, un atome d'halogène, un groupe hydroxy, trifluorométhyle, phénoxy et benzyloxy, ou R représente un groupe de formule 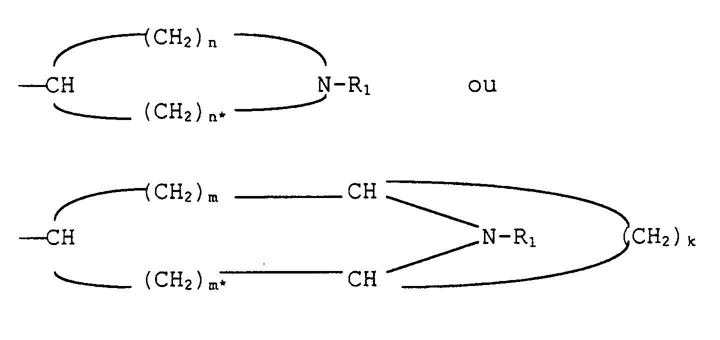 dans laquelle R₁ représente un groupe -CHO, -CO₂R₂, -CN ou -C(=O)-R₂, où R₂ représente un groupe alkyle en C₁-C₆ ou aryle, le terme "aryle" étant tel que défini ci-dessus, k, m, m*, n et n* étant indépendamment les uns des autres des entiers compris entre 1 et 4 et R₃ représentant un atome d'hydrogène, un groupe alkyle en C₁-C₆ ou aryle-alkyle en C₁-C₆, le terme "aryle" étant tel que défini ci-dessus, sous réserve que R ne soit pas un atome d'hydrogène quand R₃ représente un groupe alkyle en C₁-C₆ ou aryle-alkyle en C₁-C₆.

11. Composé selon la revendication 10, dans lequel R représente un atome d'hydrogène, un groupe alkyle en C₁-C₆ ou un groupe de formule 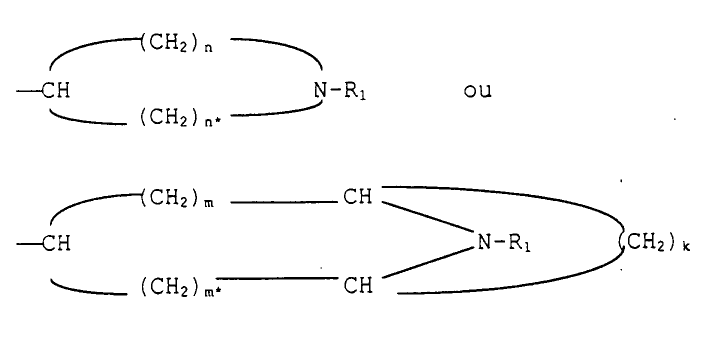 et R₃ représente un groupe benzyle.

12. Composé selon la revendication 11, dans lequel n et n* sont tous les deux l'entier 2, m et m* sont tous les deux l'entier 1, k est l'entier 2 et R₁ est un groupe -CHO ou -CN.

13. Procédé pour la préparation d'un composé tel que défini dans la revendication 10, qui comprend l'étape de faire réagir le composé 2,4-difluorophénacyle de formule II 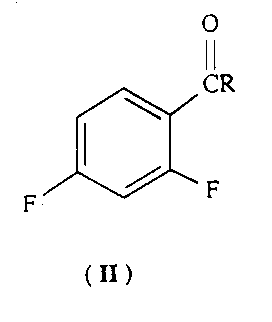 dans laquelle R est tel que défini, avec R₃SH, R₃ étant tel que défini, pourvu que R ne soit pas un atome d'hydrogène quand R₃ est un groupe alkyle en C₁-C₆ ou aryle-alkyle en C₁-C₆.

## Revendications (Revendications pour l'(les) Etat(s) contractant(s) suivant(s): ES)

1. Procédé pour la préparation de 6-fluoro-1,2-benzisothiazoles de formule I 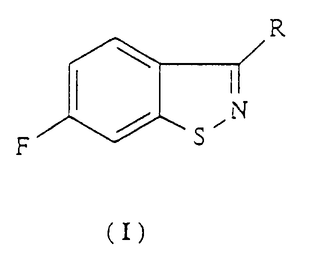 dans laquelle R représente un atome d'hydrogène, un groupe alkyle en C₁-C₆, cycloalkyle en C₃-C₇, aralkyle en C₁-C₆, aryle, le terme aryle représentant un groupe phényle éventuellement substitué avec 1, 2 ou 3 substituants choisis parmi : le groupe alkyle en C₁-C₆, alkoxy en C₁-C₆, un atome d'halogène, le groupe hydroxy, trifluorométhyle, phénoxy et benzyloxy, ou R représente un groupe de formule 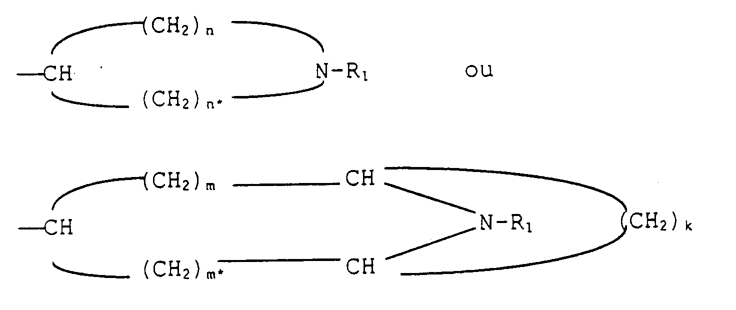 dans laquelle R₁ représente un groupe -CHO, -CO₂R₂, -CN ou -C(=O)R₂; R₂ représente un groupe alkyle en C₁-C₆ ou aryle, le terme "aryle" étant tel que défini ci-dessus; k, m, m*, n et n* étant indépendamment des entiers compris entre 1 et 4;
lequel procédé comprend
a) l'étape consistant à faire réagir un composé de formule III 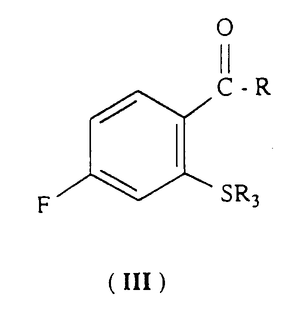 dans laquelle R est tel que défini ci-dessus et R₃ est un atome d'hydrogène, un groupe alkyle en C₁-C₆ ou aryle-alkyle en C₁-C₆, le terme "aryle" étant tel que défini ci-dessus, avec un agent d'halogénation pour obtenir un composé de formule IV 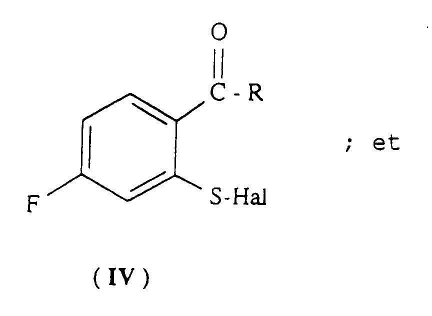 dans laquelle R est tel que défini et Hal représente un atome d'halogène, et
b) l'étape consistant à faire réagir le composé de formule IV tel qu'obtenu à l'étape a) avec l'ammoniac.

2. Procédé selon la revendication 1, dans lequel R est un atome d'hydrogène, un groupe alkyle en C₁-C₆ ou un groupe de formule 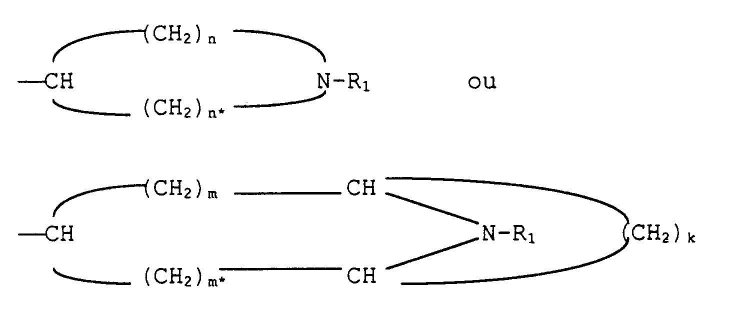 dans laquelle k, m, m*, n, n*, R₁ et R₂ sont tels que définis dans la revendication 1 et R₃ est un groupe benzyle.

3. Procédé selon la revendication 2, dans lequel R un groupe de formule 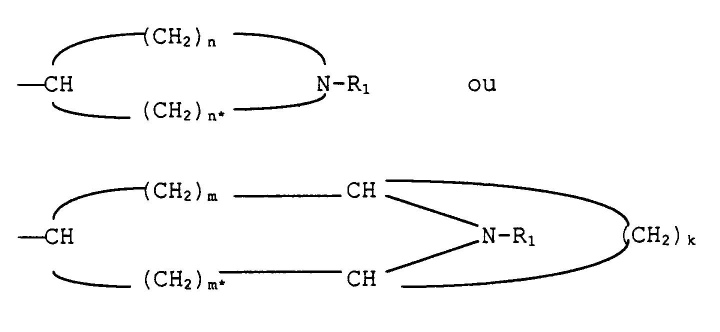 dans laquelle k, m, m*, n, n*, R₁ et R₂ sont tels que définis.

4. Procédé selon la revendication 3, dans lequel n et n* sont tous les deux l'entier 2, m et m* sont tous les deux l'entier 1, k est l'entier 2 et R₁ est un groupe -CHO ou -CN.

5. Procédé selon la revendication 1, dans lequel on fait réagir le composé de formule III avec le chlorure de sulfuryle, le bromure de sulfuryle, le brome ou le chlore comme agent d'halogénation à une température comprise entre 0°C et 100°C.

6. Procédé selon la revendication 5, dans lequel l'agent d'halogénation est le chlorure de sulfuryle et la température de réaction est comprise entre 15°C et 80°C.

7. Procédé selon la revendication 1, dans lequel on fait réagir le composé de formule IV avec l'ammoniac à une température comprise entre 0°C et 100°C.

8. Procédé selon la revendication 7, dans lequel la température de réaction est comprise entre 15°C et 80°C.

9. Procédé selon la revendication 8, dans lequel la température de réaction est la température ambiante.

10. Procédé pour la préparation d'un composé de formule III 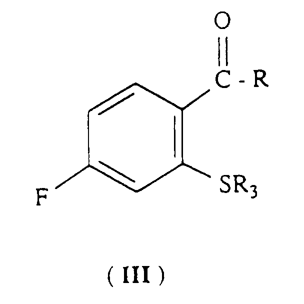 dans laquelle R représente un atome d'hydrogène, un groupe alkyle en C₁-C₆, cycloalkyle en C₃-C₇, aralkyle en C₁-C₆, aryle, le terme aryle représentant un groupe phényle éventuellement substitué avec 1, 2 ou 3 substituants choisis parmi : un groupe alkyle en C₁-C₆, alkoxy en C₁-C₆, un atome d'halogène, un groupe hydroxy, trifluorométhyle, phénoxy et benzyloxy, ou R représente un groupe de formule 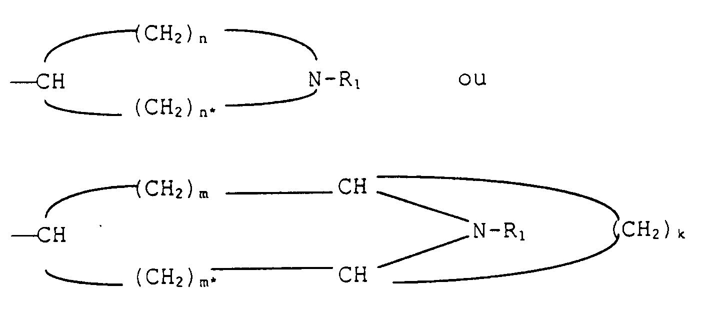 dans laquelle R₁ représente un groupe -CHO, -CO₂R₂, -CN ou -C(=O)-R₂, où R₂ représente un groupe alkyle en C₁-C₆ ou aryle, le terme "aryle" étant tel que défini ci-dessus, k, m, m*, n et n* étant indépendamment les uns des autres des entiers compris entre 1 et 4 et R₃ représentant un atome d'hydrogène, un groupe alkyle en C₁-C₆ ou aryle-alkyle en C₁-C₆, le terme "aryle" étant tel que défini, qui comprend la réaction d'un composé 2,4-difluorophénacyle de formule II 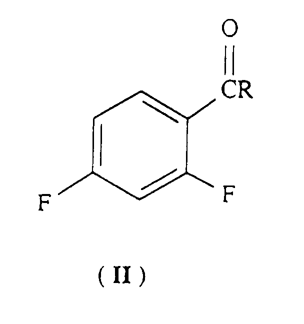 dans lequel R est tel que défini, avec R₃SO₃H, R₃ étant tel que défini, pourvu que R ne soit pas un atome d'hydrogène quand R₃ représente un groupe alkyle en C₁-C₆ ou aryle-alkyle en C₁-C₆.

11. Procédé selon la revendication 10, dans lequel R représente un atome d'hydrogène, un groupe alkyle en C₁-C₆ ou un groupe de formule 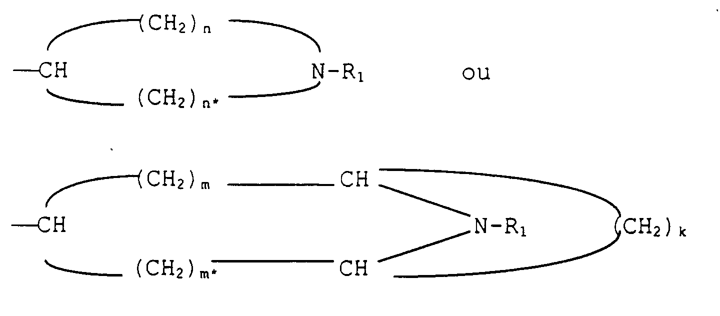 et R₃ représente un groupe benzyle.

12. Procédé selon la revendication 11, dans lequel n et n* sont tous les deux l'entier 2, m et m* sont tous les deux l'entier 1, k est l'entier 2 et R₁ est un groupe -CHO ou -CN.
